# EUROPÄISCHE PATENTSCHRIFT

(11) **EP 1 642 618 B1**
(45) Veröffentlichungstag und Bekanntmachung des Hinweises auf die Patenterteilung: **24.01.2007**
(21) Anmeldenummer: 05107988.7
(22) Anmeldetag: 01.09.2005
(51) Int. Cl.: A61N 5/10

(54) **Strahlentherapieanlage**
Radiotherapy device
Dispositif de radiothérapie

(30) Priorität: 30.09.2004 DE 102004048212
(43) Veröffentlichungstag der Anmeldung: 05.04.2006
(73) Patentinhaber: SIEMENS AKTIENGESELLSCHAFT, 80333 München (DE)
(72) Erfinder: Rietzel, Eike, 64289, Darmstadt (DE)

(56) Entgegenhaltungen:
- EP-A- 0 382 560
- US-A- 5 207 223
- US-A1- 2003 007 601
- US-A1- 2003 076 927

## Beschreibung

Die Erfindung bezieht sich auf eine Strahlentherapieanlage nach dem Oberbegriff des Anspruchs 1 oder 3. Als Strahlentherapieanlage wird in diesem Zusammenhang allgemein eine medizinische Anlage bezeichnet, mittels welcher ein Patient zu Therapiezwecken einer hochenergetischen Photonenstrahlung, d.h. einer elektromagnetischen Strahlung (Röntgenstrahlung, Gammastrahlung) oder einer Partikelstrahlung (Elektronen, Protonen, Karbonionen, etc.) ausgesetzt wird.

Im Zuge einer Strahlentherapie ist eine präzise Patentienpositionierung essentiell, um sicherzustellen, dass die zu bestrahlende Körperregion des Patienten, insbesondere ein zu bestrahlender Tumor, einer hinreichend hohen Strahlendosis ausgesetzt ist, gesundes Gewebe des Patienten aber andererseits möglichst wenig geschädigt wird. Zum Zweck der Positionierung wird üblicherweise in regelmäßigen Zeitabständen eine Lokalisierung des zu bestrahlenden Körperbereichs im Körper des Patienten vorgenommen. Diese erfolgt im Allgemeinen mittels bildgebender Röntgenverfahren, insbesondere mittels Computertomographie. Um eine Fehlpositionierung des Patienten sicher zu vermeiden, wird eine derartige Untersuchung bevorzugt direkt in Bestrahlungsposition vorgenommen.

Für Photonen-Strahlentherapie ist zu diesem Zweck aus der EP 0 382 560 A1 eine Strahlentherapieanlage bekannt, bei welcher ein Röntgenstrahlung emittierender Therapiestrahlerzeuger gleichzeitig als Teil eines Bildsystems herangezogen ist. Dem Therapiestrahlerzeuger ist dabei ein Röntgendetektor in Strahlrichtung gegenübergestellt. Die von dem Strahlerzeuger emittierte Röntgenstrahlung wird teilweise derart abgeschwächt, dass sie eine vergleichsweise geringe, aber für Bildgebungszwecke hinreichende Strahlungsintensität aufweist. Diese abgeschwächte Strahlung wird aufgenommen und zur Lokalisierung des zu bestrahlenden Körperbereichs bildgebungstechnisch ausgewertet. Gleichzeitig wird ein Strahlausschnitt mit wesentlich höherer Strahlungsintensität als Therapiestrahl auf den zu bestrahlenden Körperbereich appliziert.

Des Weiteren ist mitunter ein röntgentomographisches Bildsystem direkt an einer einen Therapiestrahlauslass rotierbar halternden Gantry angebracht. Die für tomographische Bildgebung erforderliche Rotation des Bildsystems um den Patienten erfolgt hierbei zusammen mit der Gantry-Rotation. Infolge der üblicherweise vergleichsweise geringen Rotationsgeschwindigkeit der Gantry einer Strahlentherapieanlage ist diese Aufnahmetechnik jedoch vergleichsweise zeitaufwändig.

Aus den Dokumenten US 2003/0048868 A1, DE 102 31 630 A1, US 6,307,914 B1 und US 5,207,233 A ist jeweils bekannt, einer Strahlentherapieanlage zwei Röntgenbildsysteme zur Lokalisierung des zu bestrahlenden Körperbereichs zuzuordnen, deren jeweilige Bildachsen gekreuzt zueinander ausgerichtet sind. Gemäß US 5,207,223 A sind die Bildsysteme zusammen mit einem Linearbeschleuniger als Therapiestrahlerzeuger gemeinsam an einer Gantry gehaltert.

Eine gattungsgemäße Strahlentherapieanlage ist aus US 2003/0076927 A1 bekannt. In einer Ausführung dieser Anlage sind an einer Gantry zwei oder mehr Datendetektionssysteme mit jeweils einem Röntgenstrahler und einem diesem gegenübergestellten Röntgendetektor gekreuzt zueinander angeordnet. Die Anlage ist dabei derart ausgebildet, dass der Röntgenstrahler eines Datendetektionssystems zur Erzeugung eines Therapiestrahls, d.h. als Therapiestrahlauslass verwendbar ist.

Der Erfindung liegt die Aufgabe zugrunde, eine Strahlentherapieanlage anzugeben, bei welcher eine besonders präzise und schnelle Patientenpositionierung ermöglicht ist.

Diese Aufgabe wird unabhängig gelöst durch zwei Varianten der Erfindung der Erfindung mit den Merkmale des Anspruch 1 bzw. des Anspruchs 3.

Gemäß beider Varianten der Erfindung umfasst die Strahlentherapieanlage eine Gantry, d.h. ein um eine isozentrische Achse rotierbares Traggestell. Die Gantry haltert einen Therapiestrahlauslass, der eine in der Regel auf die isozentrische Achse hin ausgerichtete Strahlachse definiert. An der Gantry sind nun erfindungsgemäß eine erste und mindestens eine weitere Bildeinheit angebracht. Jede Bildeinheit umfasst in Gegenüberstellung entlang einer Bildachse einen Röntgenstrahler und einen Röntgendetektor. Beide Bildeinheiten sind durch die Gantry-Rotation um die isozentrische Achse rotierbar. Die Bildachsen zweier Bildeinheiten sind hierbei im umgebenden Raum bzw. bezüglich eines in Bestrahlungsposition gelagerten Patienten stets unterschiedlich orientiert.

Die Aufnahme von Röntgenprojektionen, d.h. zweidimensionalen Röntgenbildern, zur Patientenpositionierung erfolgt somit erfindungsgemäß während derselben, durch die Gantry vorgegebene Rotationsbewegung mittels zweier oder mehrerer Bildeinheiten. Dadurch werden bei jeder Gantry-Position simultan Röntgenprojektionen aus unterschiedlichen Projektionsrichtungen aufgenommen. Die zur Datenaufnahme benötigte Zeit wird durch den simultanen Einsatz mehrerer Bildeinheiten erheblich reduziert, was wiederum zu einer verbesserten Ausnutzung der Strahlentherapieanlage und damit zu reduzierten Behandlungskosten pro Patient führt.

Um ein besonders großes Raumvolumen mittels der Bildeinheiten erfassen zu können, ist gemäß der ersten Variante der Erfindung gemäß Anspruch 1 der Detektor mindestens einer Bildeinheit bezüglich der zugeordneten Bildachse tangential azentrisch angeordnet, d.h. in oder gegen Rotationsrichtung der Gantry derart versetzt, dass ein entlang der Bildachse emittierter Röntgenstrahl außermittig auf dem Detektor auftrifft. Bevorzugt sind die Detektoren mindestens zweier Bildeinheiten hierbei entgegengesetzt zueinander versetzt. Ist beispielsweise also der Detektor der ersten Bildeinheit in Rotationsrichtung versetzt, so ist der Detektor der zweiten Bildeinheit entgegen der Rotationsrichtung versetzt oder umgekehrt. Durch diese Anordnung wird bei vergleichsweise großem Aufnahmevolumen gleichzeitig eine vergleichsweise kurze Aufnahmezeit erzielt, zumal sich die Asymmetrie der Detektoranordnung durch den entgegengesetzten Versatz der beiden Detektoren kompensiert, und die gesamte Rauminformation schon bei einer Gantrydrehung um einen 360° wesentlich unterschreitenden Winkel erhalten wird.

Bei der zweiten Variante der Erfindung gemäß Anspruch 3 ist vorgesehen, dass die Bildachsen mindestens zweier Bildeinheiten zueinander axial bezüglich der isozentrischen Achse versetzt sind. Durch diese Anordnung wird während einer Gantry-rotation ein entlang der isozentrischen Achse besonders gro-ßer Axialbereich des Patientenkörpers simultan erfasst, was wiederum zu einer wesentlichen Verkürzung der Aufnahmezeit beiträgt. Insbesondere sind hierbei die Bildachsen zweier Bildeinheiten bezüglich der Strahlachse entgegengesetzt versetzt, so dass die Strahlachse in Richtung der isozentrischen Achse zwischen den beiden Bildachsen angeordnet ist.

Die beiden Varianten der Erfindung sind vorteilhaft auch in Kombination miteinander einsetzbar.

In bevorzugter Ausführung sind die Bildeinheiten derart an der Gantry angebracht, dass jede zugehörige Bildachse in einer senkrecht bezüglich der isozentrischen Achse ausgerichteten Aufnahmeebene verläuft. Hierbei sind die Bildachsen je zweier Bildeinheiten in Projektion entlang der isozentrischen Achse gesehen unter einem vorgegebenen Winkelversatz angeordnet. Bevorzugt sind zwei Bildeinheiten vorgesehen. Der Winkelversatz der beiden zugehörigen Bildachsen beträgt bevorzugt zwischen 40° und 130°. In bevorzugten Ausführungen beträgt der Winkelversatz der beiden Bildachsen insbesondere etwa 90° bzw. etwa 60°.

Bevorzugt sind die Bildachsen zweier Bildeinheiten weiterhin spiegelsymmetrisch bezüglich der Strahlachse orientiert. Der Therapiestrahlauslass ist hierbei insbesondere zwischen den Detektoren der Bildeinheiten angeordnet. Insbesondere bei einer Partikel-Strahlentherapieanlage sind die Detektoren der Bildeinheiten vorteilhafterweise direkt an dem Therapiestrahlauslass angebracht.

Zweckmäßigerweise ist mindestens eine der Bildeinheiten, bevorzugt jede Bildeinheit, als Kegelstrahl(cone beam)-Bild-system ausgeführt. Hierunter wird eine tomographische Röntgenaufnahmetechnik verstanden, bei welcher der Röntgenstrahler einer Bildeinheit einen kegelförmigen Strahl emittiert, der von einem flächigen Röntgendetektor aufgenommen wird, so dass bereits bei einer Rotation der Bildeinheit um den Patienten ein Volumenbereich, und nicht lediglich eine dünne tomographische Scheibe des Patientenkörpers aufgenommen wird. Die Kegelstrahltechnik ermöglicht eine vergleichsweise schnelle Datenaufnahme ausgedehnter Körpervolumina und ist deshalb insbesondere bei einer Strahlentherapieanlage besonders geeignet.

Nachfolgend werden Ausführungsbeispiele der Erfindung anhand einer Zeichnung näher erläutert. Darin zeigen:
- FIG 1: in schematischer Draufsicht entlang einer isozentrischen Achse eine Gantry einer Strahlentherapieanlage mit einem Therapiestrahlauslass sowie mit zwei röntgentomographischen Bildeinheiten zur Patientenpositionierung,
- FIG 2: in schematischer partiell geschnittener Darstellung II-II die Gantry gemäß FIG 1 und
- FIG 3: in Darstellung gemäß FIG 2 eine Variante der Gantry gemäß FIG 1, bei welcher die beiden Bildeinheiten axial bezüglich der isozentrischen Achse zueinander versetzt angeordnet sind.

Einander entsprechende Teile und Größen sind in allen Figuren stets mit den gleichen Bezugszeichen versehen.

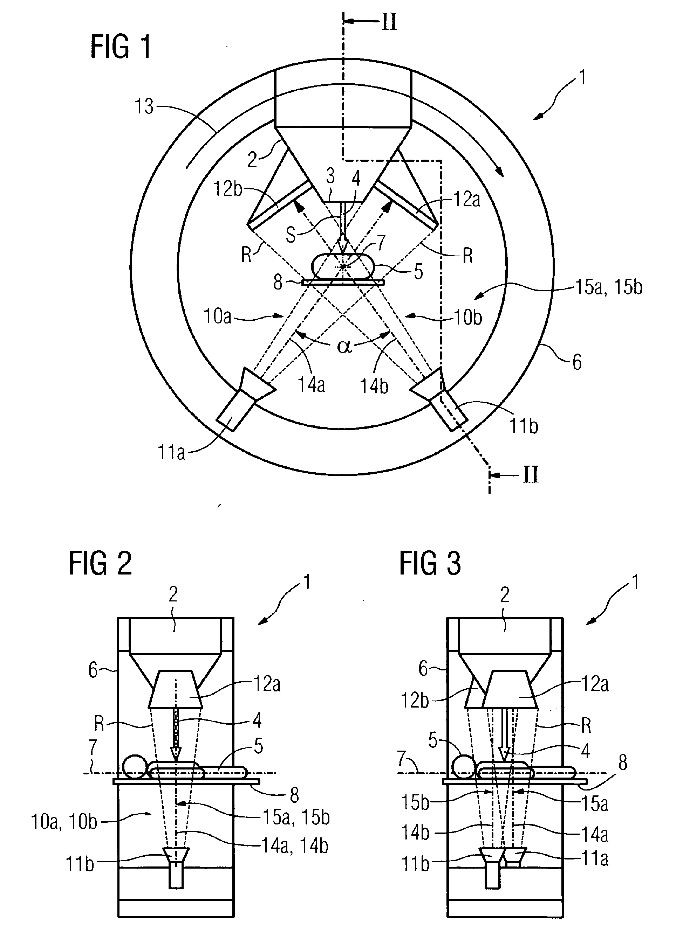

FIG 1 zeigt ausschnitthaft und schematisch eine Strahlentherapieanlage 1, nachfolgend kurz als Anlage 1 bezeichnet. Die Anlage 1 umfasst einen Therapiestrahlauslass 2, aus dessen Mündung 3 ein Therapiestrahl S entlang einer Strahlachse 4 auf den Körper eines Patienten 5 emittiert wird. Bei der Anlage 1 handelt es sich bevorzugt um eine Anlage für eine Partikel-Strahlentherapie. Der Therapiestrahlauslass 2 ist hierbei mit einem (nicht näher dargestellten) Teilchenbeschleuniger verbunden. Der Therapiestrahl S enthält in diesem Fall auf hohe Geschwindigkeit beschleunigte Teilchen, insbesondere Protonen, Karbonionen, etc.

Der Therapiestrahlauslass 2 ist an einer Gantry 6, d.h. einem im Wesentlichen ringförmigen Traggestell gehaltert. Die Gantry 6 ist um eine isozentrische Achse 7 rotierbar, so dass durch Rotation der Gantry 6 der Therapiestrahlauslass 2, und damit die Strahlachse 4 um den auf einem Patiententisch 8 etwa im Bereich der isozentrischen Achse 7 gelagerten Patienten 5 herum verschwenkbar ist.

Um einen zu bestrahlenden Körperbereich des Patienten 5 zu lokalisieren und anhand dieser Lokalisierung den Patienten 5 geeignet positionieren zu können, umfasst die Anlage 1 des Weiteren zwei Bildeinheiten 10a und 10b. Jede Bildeinheit 10a,10b umfasst einen Röntgenstrahler 11a bzw. 11b sowie einen digitalen Röntgendetektor 12a bzw. 12b. Die Röntgendetektoren 12a und 12b sind hierbei in einander entgegengesetzter Position an dem Therapiestrahlauslass 2 angebracht, so dass der Röntgendetektor 12a dem Therapiestrahlauslass 2 in einer Rotationsrichtung 13 der Gantry 6 vorgeschaltet und der Röntgendetektor 12b dem Therapiestrahlauslass 2 in Rotationsrichtung 13 nachgeschaltet ist. Die Röntgenstrahler 11a und 11b sind an der dem Therapiestrahlauslass 2 gegenüberliegenden Seite der Gantry 6 gehaltert. Der Röntgenstrahler 11a bzw. 11b einer jeden Bildeinheit 10a,10b ist entlang einer zugehörigen Bildachse 14a bzw. 14b in Gegenüberstellung zu dem jeweils zugehörigen Röntgendetektor 12a bzw. 12b angeordnet. Die Bildachsen 14a und 14b sind jeweils durch den Zentralstrahl der von dem jeweiligen Röntgenstrahler 11a bzw. 11b emittierten Röntgenstrahlung R gegeben.

Bei den Bildeinheiten 10a,10b handelt es sich um so genannte Kegelstrahl-Bildsysteme. Hierbei weist die von den Röntgenstrahlern 11a,11b emittierte Röntgenstrahlung R eine kegelartige Abstrahlungscharakteristik auf. Mit anderen Worten verbreitert sich das von jedem Röntgenstrahler 11a,11b ausgehende Strahlenbündel mit zunehmendem Abstand sowohl innerhalb einer senkrecht zur isozentrischen Achse 7 verlaufenden Aufnahmeebene 15a,15b als auch in hierzu senkrechter Richtung.

Die Bildeinheiten 10a,10b sind derart innerhalb der Gantry 6 positioniert, dass die jeweiligen Bildachsen 14a,14b im Wesentlichen radial bezüglich der isozentrischen Achse 7 ausgerichtet sind und letztere somit etwa unter einem rechten Winkel schneiden. Insbesondere verläuft jede Bildachse 14a,14b innerhalb der zugehörigen Aufnahmeebene 15a,15b. Bevorzugt sind die Bildeinheiten 10a,10b bezüglich der isozentrischen Achse 7 auf gleicher Höhe angeordnet, so dass die Aufnahmeebenen 15a und 15b zusammenfallen (FIG 2). In einer Variante der Anlage 1 gemäß FIG 3 sind die Bildeinheiten 10a,10b dagegen zueinander axial versetzt angeordnet, so dass durch die Bildachsen 14a und 14b zwei parallele und voneinander beabstandete Aufnahmeebenen 15a und 15b definiert werden. Gemäß FIG 3 sind die Bildeinheiten 10a und 10b bezüglich der Strahlachse 4 in entgegengesetzte axiale Richtungen versetzt, so dass die Strahlachse 4 etwa mittig zwischen den Aufnahmeebenen 15a und 15b angeordnet ist. Auf diese Weise wird das Aufnahmevolumen, d.h. der von den Bildeinheiten 10a und 10b bildgebungstechnisch abgetastete Raumbereich, in axialer Richtung vergrößert. Die Bildeinheiten 10a und 10b sind dabei insbesondere derart angeordnet, dass sich die Aufnahmevolumina der beiden Bildeinheiten 10a und 10b im Bereich der Strahlachse 4 überlappen.

Sowohl in der Ausführungsform gemäß FIG 2 als auch der Variante gemäß FIG 3 sind die Bildeinheiten 10a,10b derart ausgerichtet, dass sich ihre jeweiligen Bildachsen 14a und 14b in Projektion entlang der isozentrischen Achse 7 unter einem Versatzwinkel α schneiden. In der dargestellten Ausführungsform der Anlage 1 beträgt der Versatzwinkel α insbesondere aus konstruktiven Gründen etwa 60°. In einer (nicht näher dargestellten) Variante der Anlage 1 beträgt der Versatzwinkel etwa 90°.

Wie aus FIG 1 zu erkennen ist, sind die Röntgendetektoren 12a und 12b nicht zentral bezüglich der jeweiligen Bildachse 14a bzw. 14b, sondern tangential exzentrisch angeordnet. Diese Exzentrizität ist für beide Bildeinheiten 10a und 10b entgegengesetzt ausgeprägt. So ist der Röntgendetektor 12a der Bildeinheit 10a gegenüber einer mittigen Position in Rotationsrichtung 13, der Röntgendetektor 12b der Bildeinheit 10b entgegen der Rotationsrichtung 13 versetzt. Durch diese tangential exzentrische Anordnung der Röntgendetektoren 12a und 12b wird bei gegebener Detektorgröße gegenüber einer mittigen Anordnung eine Vergrößerung des Aufnahmevolumens in radialer Richtung erzielt. Durch den entgegengesetzten Versatz der beiden Bildeinheiten 10a und 10b wird die durch die exzentrische Detektoranordnung verursachte Asymmetrie der Datenaufnahme kompensiert, so dass insbesondere bereits bei einer halben Gantry-Drehung die gesamte erzielbare Volumeninformation zur Verfügung steht.

Zur Lokalisierung der zu bestrahlenden Körperregion des Patienten 5 werden aus einer Vielzahl von Projektionsrichtungen mittels der Bildeinheiten 10a und 10b zweidimensionale Röntgenbilder aufgenommen, aus welchen mittels einer nicht näher dargestellten digitalen Auswerteeinheit ein dreidimensionaler Bilddatensatz eines untersuchten Körperbereichs des Patienten 5 errechnet wird. Die Bildaufnahme erfolgt, wie bei röntgentomographischen Aufnahmen üblich, unter Rotation der Gantry 6 und der daran befestigten Bildeinheiten 10a und 10b um den Patienten 5. Die Bildaufnahme erfolgt vor Beginn der eigentlichen Bestrahlungsphase, um den zu bestrahlenden Körperbereich des Patienten 5 nach Maßgabe der Bildinformation im I-sozentrum des Therapiestrahls S justieren zu können. Die Bildaufnahme kann des Weiteren auch zu Kontrollzwecken während der Bestrahlungsphase aufrechterhalten werden.

Durch den simultanen Einsatz zweier Bildeinheiten 10a und 10b wird insbesondere eine erhebliche Verkürzung der Bildaufnahmezeit erreicht. Hierdurch verringert sich auch die Gesamtdauer der Strahlungssitzung, d.h. der Zeitspanne, während welcher der Patient 5 innerhalb der Gantry 6 in Bestrahlungsposition gelagert werden muss. Infolge der verkürzten Behandlungsdauer lässt sich wiederum ein höherer Patientendurchsatz bei der Anlage 1 erreichen, was insbesondere unter dem Gesichtspunkt der hohen Betriebskosten einer Strahlentherapieanlage einen entscheidenden Vorteil darstellt.

## Patentansprüche

1. Strahlentherapieanlage (1) mit einer Gantry (6), an welcher
- ein eine Strahlachse (4) definierender Therapiestrahlauslass (2),
- eine erste Bildeinheit (10a), die in Gegenüberstellung entlang einer ersten Bildachse (14a) einen Röntgenstrahler (11a) und einen Röntgendetektor (12a) umfasst, und
- mindestens eine weitere Bildeinheit (10b), die in Gegenüberstellung entlang einer jeweils weiteren Bildachse (14b) einen Röntgenstrahler (11b) und einen Röntgendetektor (12b) umfasst,
um eine isozentrische Achse (7) rotierbar gehaltert sind, wobei die Bildachsen (14a,14b) zweier Bildeinheiten (10a,10b) jeweils unterschiedlich orientiert sind, **dadurch gekennzeichnet, dass** der Detektor (12a,12b) zumindestens einer Bildeinheit (10a,10b) bezüglich der zugeordneten Bildachse (14a,14b) tangential exzentrisch angeordnet ist.

2. Strahlentherapieanlage (1) nach Anspruch 1,
**dadurch gekennzeichnet, dass** die Detektoren (12a,12b) zweier Bildeinheiten (10a,10b) zueinander entgegengesetzt exzentrisch bezüglich der jeweils zugehörigen Bildachse (14a,14b) angeordnet sind.

3. Strahlentherapieanlage (1) mit einer Gantry (6), an welcher
- ein eine Strahlachse (4) definierender Therapiestrahlauslass (2),
- eine erste Bildeinheit (10a), die in Gegenüberstellung entlang einer ersten Bildachse (14a) einen Röntgenstrahler (11a) und einen Röntgendetektor (12a) umfasst, und
- mindestens eine weitere Bildeinheit (10b), die in Gegenüberstellung entlang einer jeweils weiteren Bildachse (14b) einen Röntgenstrahler (11b) und einen Röntgendetektor (12b) umfasst,
um eine isozentrische Achse (7) rotierbar gehaltert sind, wobei die Bildachsen (14a,14b) zweier Bildeinheiten (10a,10b) jeweils unterschiedlich orientiert sind,
**dadurch gekennzeichnet, dass** die Bildachsen (14a,14b) mindestens zweier Bildeinheiten (10a,10b) axial bezüglich der isozentrischen Achse (7) zueinander versetzt angeordnet sind.

4. Strahlentherapieanlage (1) nach Anspruch 3,
**dadurch gekennzeichnet, dass** die Bildachsen (14a,14b) zweier Bildeinheiten (10a,10b) bezüglich der Strahlachse (4) entgegengesetzt versetzt sind.

5. Strahlentherapieanlage (1) nach einem der Ansprüche 1 bis 4,
**dadurch gekennzeichnet, dass** jede Bildachse (14a,14b) in einer auf die isozentrische Achse (7) senkrechten Aufnahmeebene (15a,15b) verläuft, wobei die erste Bildachse (14a) zu der oder jeder weiteren Bildachse (14b) in Projektion entlang der isozentrischen Achse (7) unter einem vorgegebenen Winkelversatz (α) angeordnet ist.

6. Strahlentherapieanlage (1) nach einem der Ansprüche 1 bis 5,
**dadurch gekennzeichnet, dass** der Winkelversatz (α) zwischen 40° und 130° beträgt.

7. Strahlentherapieanlage (1) nach einem der Ansprüche 1 bis 6,
**dadurch gekennzeichnet, dass** zwei Bildachsen (14a,14b) in Projektion entlang der isozentrischen Achse (7) bezüglich der Strahlachse (4) spiegelsymmetrisch orientiert sind.

8. Strahlentherapieanlage (1) nach einem der Ansprüche 1 bis 7,
**dadurch gekennzeichnet, dass** mindestens eine der Bildeinheiten (10a,10b) als Kegelstrahlbildsystem ausgeführt ist.

9. Strahlentherapieanlage (1) nach einem der Ansprüche 1 bis 8,
**dadurch gekennzeichnet, dass** der Röntgendetektor (12a, 12b) mindestens einer Bildeinheit (10a,10b) an dem Therapiestrahlauslass (2) angebracht ist.

## Claims

1. Radiotherapy system (1) with a gantry (6) on which
- a therapeutic beam outlet (2) defining a beam axis (4),
- a first imaging unit (10a), which comprises an x-ray emitter (11a) and an x-ray detector (12a) disposed opposite one another along a first imaging axis (14a), and
- at least one further imaging unit (10b), which comprises an x-ray emitter (11b) and an x-ray detector (12b) disposed opposite one another along a respectively further imaging axis (14b)
are supported in such a manner that they can be rotated about an isocentric axis (7), the imaging axes (14a, 14b) of two imaging units (10a, 10b) being oriented differently in each instance,
**characterised in that** the detector (12a, 12b) of at least one imaging unit (10a, 10b) is disposed in a tangentially eccentric manner in relation to the assigned imaging axis (14a, 14b).

2. Radiotherapy system (1) according to claim 1,
**characterised in that** the detectors (12a, 12b) of two imaging units (10a, 10b) are disposed opposite one another in an eccentric manner in relation to the respectively associated imaging axis (14a, 14b).

3. Radiotherapy system (1) with a gantry (6) on which
- a therapeutic beam outlet (2) defining a beam axis (4),
- a first imaging unit (10a), which comprises an x-ray emitter (11a) and an x-ray detector (12a) disposed opposite one another along a first imaging axis (14a), and
- at least one further imaging unit (10b), which comprises an x-ray emitter (11b) and an x-ray detector (12b) disposed opposite one another along a respectively further imaging axis (14b)
are supported in such a manner that they can be rotated about an isocentric axis (7), the imaging axes (14a, 14b) of two imaging units (10a, 10b) being oriented differently in each instance,
**characterised in that** the imaging axes (14a, 14b) of at least two imaging units (10a, 10b) are disposed in an axially offset manner in respect of one another in relation to the isocentric axis (7).

4. Radiotherapy system (1) according to claim 3,
**characterised in that** the imaging axes (14a, 14b) of two imaging units (10a, 10b) are offset in an opposing manner in relation to the beam axis (4).

5. Radiotherapy system (1) according to one of claims 1 to 4,
**characterised in that** each imaging axis (14a, 14b) extends in a recording plane (15a, 15b) perpendicular to the isocentric axis (7), the first imaging axis (14a) being disposed at a predetermined angle offset (α) in relation to the or each further imaging axis (14b) in projection along the isocentric axis (7).

6. Radiotherapy system (1) according to one of claims 1 to 5,
**characterised in that** the angle offset (α) is between 40° and 130°.

7. Radiotherapy system (1) according to one of claims 1 to 6,
**characterised in that** two imaging axes (14a, 14b) are oriented with mirror symmetry in relation to the beam axis (4) in projection along the isocentric axis (7).

8. Radiotherapy system (1) according to one of claims 1 to 7,
**characterised in that** at least one of the imaging units (10a, 10b) is embodied as a cone beam imaging system.

9. Radiotherapy system (1) according to one of claims 1 to 8,
**characterised in that** the x-ray detector (12a, 12b) of at least one imaging unit (10a, 10b) is mounted on the therapeutic beam outlet (2).

## Revendications

1. Appareillage de thérapie par rayonnement (1) comprenant un portique ou gantry (6) sur lequel sont supportés de manière à pouvoir tourner autour d'un axe isocentrique (7),
- un élément de sortie (2) de rayonnement de thérapie définissant un axe de rayonnement (4),
- une première unité d'image (10a), qui comprend un émetteur de rayons X (11a) et un détecteur de rayons X (12a) agencés en opposition le long d'un premier axe d'image (14a), et
- au moins une autre unité d'image (10b), qui comprend un émetteur de rayons X (11b) et un détecteur de rayons X (11a) agencés en opposition le long d'un autre axe d'image respectif (14b),
les axes d'image (14a, 14b) de deux unités d'image (10a, 10b) étant orientés respectivement de manière différente,
**caractérisé en ce que** le détecteur (12a, 12b) d'au moins une unité d'image (10a, 10b) est agencé de manière tangentiellement excentrée par rapport à l'axe d'image (14a, 14b) associé.

2. Appareillage de thérapie par rayonnement (1) selon la revendication 1, **caractérisé en ce que** les détecteurs (12a, 12b) de deux unités d'image (10a, 10b) sont agencés de manière excentrée mutuellement opposée par rapport à l'axe d'image (14a, 14b) respectivement associé.

3. Appareillage de thérapie par rayonnement (1) comprenant un portique ou gantry(6) sur lequel sont supportés de manière à pouvoir tourner autour d'un axe isocentrique (7),
- un élément de sortie (2) de rayonnement de thérapie définissant un axe de rayonnement (4),
- une première unité d'image (10a), qui comprend un émetteur de rayons X (11a) et un détecteur de rayons X (12a) agencés en opposition le long d'un premier axe d'image (14a), et
- au moins une autre unité d'image (10b), qui comprend un émetteur de rayons X (12b) et un détecteur de rayons X (11a) agencés en opposition le long d'un autre axe d'image respectif (14b),
les axes d'image (14a, 14b) de deux unités d'image (10a, 10b) étant orientés respectivement de manière différente,
**caractérisé en ce que** les axes d'image (14a, 14b) d'au moins deux unités d'image (10a, 10b) sont agencés de manière axialement décalée l'un par rapport à l'autre par rapport à l'axe isocentrique (7).

4. Appareillage de thérapie par rayonnement (1) selon la revendication 3, **caractérisé en ce que** les axes d'image (14a, 14b) de deux unités d'image (10a, 10b) sont décalés de manière mutuellement opposée par rapport à l'axe de rayonnement (4).

5. Appareillage de thérapie par rayonnement (1) selon l'une des revendications 1 à 4, **caractérisé en ce que** chaque axe d'image (14a, 14b) s'étend dans un plan de prise de vue (15a, 15b) perpendiculaire à l'axe isocentrique (7), le premier axe d'image (14a) étant agencé selon un décalage angulaire (α) prédéterminé par rapport à l'autre ou chaque autre axe d'image (14b), en projection le long de l'axe isocentrique (7).

6. Appareillage de thérapie par rayonnement (1) selon l'une des revendications 1 à 5, **caractérisé en ce que** le décalage angulaire (α) est compris entre 40° et 130°.

7. Appareillage de thérapie par rayonnement (1) selon l'une des revendications 1 à 6, **caractérisé en ce que** deux axes d'image (14a, 14b), en projection le long de l'axe isocentrique (7) sont orientés de manière symétrique comme dans un miroir par rapport à l'axe de rayonnement (4).

8. Appareillage de thérapie par rayonnement (1) selon l'une des revendications 1 à 7, **caractérisé en ce que** l'une au moins des unités d'image (10a, 10b) est réalisée en tant que système d'image à rayonnement en forme de cône.

9. Appareillage de thérapie par rayonnement (1) selon l'une des revendications 1 à 8, **caractérisé en ce que** le détecteur de rayons X (12a, 12b) d'au moins une unité d'image (10a, 10b) est placé sur l'élément de sortie (2) du rayonnement de thérapie.
